# EUROPEAN PATENT SPECIFICATION

(11) **EP 0 444 081 B1**
(45) Date of publication and mention of the grant of the patent: **12.05.1999**
(21) Application number: 89912690.8
(22) Date of filing: 17.11.1989
(51) Int. Cl.: A61K 9/22, A61K 47/00, A61K 38/00, A61K 38/27, A61L 27/00

(54) **PHARMACEUTICAL PREPARATION**
ARZNEIMITTELZUBEREITUNG
PREPARATION PHARMACEUTIQUE

(30) Priority: 17.11.1988 SE 8804164
(43) Date of publication of application: 04.09.1991
(73) Proprietor: PRISELL, Per, 118 67 Stockholm (SE); NORSTEDT, Gunnar, 161 42 Bromma (SE)
(72) Inventor: PRISELL, Per, 118 67 Stockholm (SE); NORSTEDT, Gunnar, 161 42 Bromma (SE)
(74) Representative: Bergvall, Stina-Lena
(86) International application number: SE8900666
(87) International publication number: WO9005522

(56) References cited:
- EP-A- 0 014 995
- EP-A- 0 103 184
- WO-A-88/07078
- WO-A-88/09818
- WO-A-89/04838
- US-A- 4 469 681
- US-A- 4 687 808
- US-A- 4 828 563

## Description

The present invention is relating to pharmaceutical preparations consisting of one or several carriers in combination with one or several receptors/binding proteins. The receptors/binding proteins are connected to suitable ligands, e.g. growth hormone.
By this combination; carrier+receptor/binding protein+e.g. active peptide, according to the invention, a slow release preparation is obtained. Reversely, the principles according to this invention might also be useful in the situation of abnormal, increased production of growth factors, e.g. tumour growth, this time carrier+receptor/binding protein acting as a selective resorption agent of growth factors.

Receptors are protein molecules that can bind hormones and growth factors (=ligands). Each receptor type is specific for its ligand. The receptor function is to convey external e.g. hormonal signals into the target cell. Novel achievements in receptor research have resulted in the possibility to obtain large quantities of specific pure receptor protein. This possibility is fundamental for the present invention which hereby defines a novel therapeutical role of receptors. Some of the known receptors can be exemplified by : Insulin-like growth factor-1-receptor, Insulin-like growth factor-2-receptor, Insulin-receptor, Platelet derived growth factor-receptor, Fibroblast growth factor-receptor, Epidermal growth factor-receptor, Nerve growth factor-receptor, Colony stimulating factor-receptor, Transforming growth factor-receptors, Growth hormone-receptor, Parathyroid hormone-receptor, Calcitonin-receptor, Estrogen-receptor, Tumour necrosis factor-receptor, Insulin-like growth factor serum binding protein and Corticosteroid binding globulin.

It is generally known that growth factors and hormones, both in animals and in humans stimulates important cellular processes concerning cell division, growth, maturation, differentiation. In addition healing and regenerative processes are also regulated by these factors. The growth factors/hormones comprise e.g. Insulin-like growth factor-1 and 2; IGF-1, IGF-2, Platelet derived growth factor; PDGF, Epidermal growth factor; EGF, Fibroblast growth factor; FGF, Nerve growth factor; NGF, Colony stimulating factor; CSF, Transforming growth factor; TGF, Tumour necrosis factor; TNF, Calcitonin; CT, Parathyroid hormone; PTH, Growth hormone; GH, Estrogens, Bombesin, Bone morphogenetic protein; BMP, Insulin and Corticosteroids.
Insulin, estrogens, corticosteroids, CT and GH are all well known pharmaceutical agents in the daily clinical practice. The research is intense concerning the other different previously mentioned growth factors and hormones. Relating to various studies and results e.g. PDGF and IGF-1 potentiates wound healing, GH increases fracture healing etc. Although most of these growth factors and hormones do have interesting effects according to different recorded data, clinical implications are yet to be found. The results as hitherto obtained indicates that the ways of administration, as presently used, restrict the use of the substances as drugs, e.g. due to the short half-life of peptides, the potency and the potential toxicity of the substances. By the present invention, offering slow release, the bioactivity of the peptides is prolonged by its connection to the carrier materials. The carriers, such as hyaluronic acid, may also have favourable biological effects per se and e.g. a polylactide rod may except being the stabilizing osteosynthetic material also act as a slow release carrier distributing fracture healing promoting polypeptides.

Hammonds et al demonstrate in WO 88/09818 that the extracellular part of the GB receptor/GH bindning protein (GH-BP) can be produced in E. coli and purified from bacterial extracts. In addition it was shown that GH bound to GH-BP resulted in enhanced in vivo stability and efficacy of GH, which is confirmatory to a previous study of Bauman et al (J. Clin. Endocrinol. Metab. 64:657-660 (1987)). The GB-BP bound GH had a significantly longer half-life in vivo compared to unbound GH. The present invention differs from this principle in that according to the invention not only ligand-receptor interactions are used but also the ligand bound to receptors are immobilised into carrier materials and thus offering a new type of slow release.

The present invention thus involves three components; carriers, receptors and ligands. The knowledge of each of these components is extensive (for textbook information see: Ch 12 and 14 in "The molecular biology of the cell", Alberts et al., Garland pub. inc. NY and London; 1989). However, to a man skilled in the art, not even with knowledge based on this information it is clear that a slow release will occur for the peptides described above, if a pharmaceutical preparation is prepared according to the invention.
1. Cross-linking between a matrix gel e.g. hyaluronic acid and a receptor protein can be achieved by a variety of means, e.g. via imidocarbonates, carbonates, oxiranes, aziridines and activated double bonds and halogens. Such strategies have previously been used to immobilize enzymes on polysaccaride gels.
2. By definition a hormone or growth factor-receptor specifically binds its ligand by high affinity and in a reversible manner and the rate of association is much faster than the dissociation rate. These receptor characteristics, high affinity, high specificity and reversibility are thus obligatory in order to use the term receptor. The dissociation rate is usually determined by incubating the receptor with radiolabelled ligands until equilibrium is reached. Excess unlabelled ligand is added and the release of non-receptorbound radioactivity measured as a function of time reveals the dissociation rate. From the above stated, it should be clear that the combination of a carrier, with a receptor, with a ligand gives, as a general accepted principle, a slow release of the ligand. The present invention introduce receptors in a therapeutical context, in addition it defines carriers allowing slow release.
Below is a schematic practical outline described, revealing the principles of the manufacturing of a pharmaceutical preparation according to this invention.
Example: the preparation of a slow release GH-complex.
a. Isolation of the extracellular domain of the GH receptor is achieved using a part of GH receptor cDNA put into an expression vector- driven by the MT promotor. Purification of this truncated receptor from media of transfected cells is achieved by affinity chromatography (using hGH Sepharose®) followed by a size separating gel chromatography.
b. Hyaluronic acid is commercially available and is linked to the purified receptor by the use of the crosslinking agent, see above. The high molecular weight complex is purified from remaining GH receptor by repeated centrifugations. Around 75% of crosslinked receptor is functionally intact.
c. The crosslinked hyaluronic acid-GH receptor preparation is then incubated with excess levels of GH for 2 hours at 37 C, unbound hormone is removed by centrifugation.
d. The preparation will, when injected e.g. subcutaneously , slowly release GH in a dose dependent way, not only based upon the amount of GH, but also according to the number of GH-receptors coupled to the gel. The rate of dissociation for a given preparation is determined for each batch by testing the release of immunoreactive GH in vitro (or by testing the release of radioactive GH in animal models).

A. An experiment measuring the increased body weight subsequent to different types of GH-treatment in a group of hypophysectomised (HX) rats was performed. Three HX rats were subcutaneously injected each with a hyaluronic acid-GH-receptor-GH preparation, according to the invention, containing approx 1.2 mg receptor bound GH. Three HX rats subcutaneously injected by the same amount of GH in a water solution served as controls. The slow release treated group had a significant increased bodyweight lasting more than 4 days compared to the controls.
B. A group of rats were injected in the tail by either 6 mikrograms IGF-1+IGF-1 binding protein+Hyaluronic acid, or 6 mikrograms IGF-1 in a water solution. The IGF-1 as used contained trace amounts (200 000 cpm) of radiolabelled IGF-1. Three rats in each group were used. After different time points the end of the tail was amputated and radioactivity analyzed. The first group of rats were found to release IGF-1 significantly slower (more than 2 days) than the controls.

### Biodegradable polymers.

Examples of biodegradable polymers are listed below:
Polyglycolide (PGA)
Copolymers of glycolide:
Glycolide/L-lactide copolymers (PGA/PLLA)
Glycolide/trimethylene carbonate copolymers (PGA/TMC)

Polylactides (PLA)
Stereo-copolymers of PLA:
Poly-L-lactide (PLLA)
Poly-DL-lactide (PDLLA)
L-lactide/DL-lactide copolymers
Copolymers of PLA:
Lactide/tetramethylglycolide copolymers
Lactide/trimethylene carbonate copolymers
Lactide / ∂-valerolactone copolymers
Lactide/ε-caprolactone copolymers
PLA/polyethylene oxide copolymers
unsymmetrical 3,6-substituted poly-1,4-dioxane-2,5-diones

Poly-β-hydroxybutyrate (PHBA)
PHBA/β-hydroxyvalerate copolymers (PHBA/HVA)

Poly-p-dioxanone (PDS)
Poly-∂-valerolactone
Poly-ε-caprolactone
Methylmethacrylate-N-vinyl pyrrolidine copolymers
Polyesteramides
Polyesters of oxalic acid
Polydihydropyranes
Polyalkyl-2-cyanoacrylates
Polyurethanes (PU)

Polyvinylalcohol (PVA)
Poly-β-malic acid (PMLA)
Poly-β-alcanoic acids.

Within the body all of these are degraded by hydrolysis. The different polymers varies in their structural and chemical aspects which gives them different strength, action, degradation-time and usefulness. PDS for instance is used as a resorptive suture-material. The PGA is used in osteosynthetic material as rods, plates and screws e.g. the Biofix®. PLLA ligaments are used, in research, as replacement for the anterior cruciate ligament of the knee etc.
A combination of the different principles, i.e. the stabilizing osteosynthetic material in combination with a local slow release of e.g. fracture-healing promoting peptides, is attractive. The biodegradable polymers structurally being porous and having different grade of coating facilitates the absorption of the growth factor+receptor complex.
C. In an experiment, according the principles above, PGA rods (1.5 x 8 mm) were "loaded" with radiolabelled IGF-1+IGF-1 binding protein (see p. B) using vacuum forces, end parts sealed by melting. The rods were implanted in the end of the tail in three rats. Three control rats were injected in the same part of the tail using the same amount of radiolabelled IGF-1 in a water solution. The slow release rats, according to the invention, had a significant higher radioactivity in tails compared to the controls.

## Claims

1. Pharmaceutical preparation for use in vivo to achieve slow release of growth factors and hormones, characterized in that it is a combination of: one or several receptor/binding proteins, for binding growth factors or hormones, selected from the group, consisting of
Insulin-like growth factor-1-receptor;
Insulin-like growth factor-2-receptor;
Insulin-receptor;
Platelet derived growth factor-receptor;
Fibroblast growth factor-receptor;
Epidermal growth factor-receptor;
Nerve growth factor-receptor;
Colony stimulating factor-receptor;
Transforming growth factor-receptors;
Growth hormone-receptor;
Parathyroid hormone-receptor;
Calcitonin-receptor;
Estrogen-receptor;
Tumour necrosis factor-receptor;
Insulin-like growth factor serum binding protein;
Corticosteroid binding globulin;
and hyaluronic acid, or its derivatives, or a biodegradable polymer, selected from the group consisting of
Polyglycolide (PGA);
Copolymers of glycolide such as
Glycolide/L-lactide copolymers (PGA/PLLA)
Glycolide/trimethylene carbonate copolymers (PGA/TMC);
Polylactides (PLA);
Stereo-copolymers of PLA such as
Poly-L-lactide (PLLA)
Poly-DL-lactide (PDLLA)
L-lactide/DL-lactide copolymers;
Copolymers of PLA such as
Lactide/tetramethylglycolide copolymers
Lactide/trimethylene carbonate copolymers
Lactide / ∂-valerolactone copolymers
Lactide/ε-caprolactone copolymers;
PLA/polyethylene oxide copolymers;
Unsymmetrical 3,6-substituted poly-1,4-dioxane-2,5-diones;
Poly-β-hydroxybutyrate (PHBA);
PHBA/β-hydroxyvalerate copolymers (PHBA/HVA);
Poly-p-dioxanone (PDS);
Poly-∂-valerolactone;
Poly-ε-caprolactone;
Methylmethacrylate-N-vinyl pyrrolidine copolymers;
Polyesteramides;
Polyesters of oxalic acid;
Polydihydropyranes;
Polyalkyl-2-cyanoacrylates;
Polyurethanes (PU);
Polyvinylalcohol (PVA);
Poly-β-malic acid (PMLA);
Poly-β-alcanoic acids;
in combination, with the ligands to the receptor/binding proteins, and
that the receptors/binding proteins are mixed, crosslinked with or covalently bound to the hyaluronic acid or the biodegradable polymer.

2. Pharmaceutical preparation, according to claim 1, characterized in that the ligands e.g. are:
Insulin-like growth factor-1 and 2 (IGF-1, IGF-2);
Platelet derived growth factor (PDGF);
Epidermal growth factor (EGF);
Fibroblast growth factor (FGF);
Nerve growth factor (NGF);
Colony stimulating factor (CSF);
Transforming growth factor (TGF);
Tumour necrosis factor (TNF);
Calcitonin (CT);
Parathyroid hormone (PTH);
Growth hormone (GH);
Estrogens;
Bombesin;
Bone morphogenetic protein (BMP);
Corticosteroids;
Insulin.

## Patentansprüche

1. Pharmazeutische Zubereitung zur Verwendung in vivo zum Erreichen einer verzögerten Freisetzung von Wachstumsfaktoren und Hormonen, **dadurch gekennzeichnet**, daß sie eine Kombination ist von: einem oder mehreren Rezeptor/Bindungsproteinen, zum Binden von Wachstumsfaktoren oder Hormonen, ausgewählt aus der Gruppe bestehend aus:
Insulin-ähnlicher Wachstumsfaktor 1-Rezeptor;
Insulin-ähnlicher Wachstumsfaktor 2-Rezeptor;
Insulin-Rezeptor;
Plättchen-entstammender Wachstumsfaktor-Rezeptor;
Fibroblasten-Wachstumsfaktor-Rezeptor;
epidermaler Wachstumsfaktor-Rezeptor;
Nerven-Wachstumsfaktor-Rezeptor;
Kolonie-stimulierender Faktor-Rezeptor;
transformierender Wachstumsfaktor-Rezeptoren;
Wachstumshormon-Rezeptor;
Parathyreoidhormon-Rezeptor;
Calcitonin-Rezeptor;
Östrogen-Rezeptor;
Tumor-Nekrose-Faktor-Rezeptor;
Insulin-ähnlicher Wachstumsfaktor-Setumbindungsprotein;
Corticosteroid-Bindungsglobulin;
und Hyaluronsäure, oder ihren Derivaten oder einem biologisch abbaubaren Polymer, ausgewählt aus der Gruppe bestehend aus:
Polyglykolid (PGA);
Copolymeren von Glykolid wie
Glykolid/L-Lactid-Copolymeren (PGA/PLLA)
Glykolid/Trimethylencarbonat-Copolymeren (PGA/TMC);
Polylactiden (PLA);
Stereo-Copolymeren von PLA wie
Poly-L-lactid (PLLA)
Poly-DL-lactid (PDLLA)
L-Lactid/DL-Lactid-Copolymeren;
Copolymeren von PLA wie
Lactid/Tetramethylglykolid-Copolymeren
Lactid/Trimethylencarbonat-Copolymeren
Lactid/δ-Valerolacton-Copolymeren
Lactid/ε-Caprolacton-Copolymeren;
PLA/Polyethylenoxid-Copolymeren;
unsymmetrischen 3,6-substituierten Poly-1,4-dioxan-2,5-dionen;
Poly-β-hydroxybutyrat (PHBA);
PHBA/β-Hydroxyvalerat-Copolymeren (PHBA/HVA);
Poly-p-dioxanon (PDS);
Poly-δ-valerolacton;
Poly-ε-caprolacton;
Methylmethacrylat-N-Vinylpyrrolidin-Copolymeren;
Polyesteramiden;
Polyestern von Oxalsäure;
Polydihydropyranen;
Polyalkyl-2-cyanoacrylaten;
Polyurethanen (PU);
Polyvinylalkohol (PVA);
Poly-β-hydroxybernsteinsäure (PMLA);
Poly-β-alkansäuren;
in Kombination, mit den Liganden zu den Rezeptor/Bindungsproteinen, und daß die Rezeptoren/Bindungsproteine mit der Hyaluronsäure oder dem biologisch abbaubaren Polymer vermischt, vernetzt oder daran kovalent gebunden sind.

2. Pharmazeutische Zubereitung nach Anspruch 1, **dadurch gekennzeichnet**, daß die Liganden beispielsweise sind:
Insulin-ähnlicher Wachstumsfaktor 1 und 2 (IGF-1, IGF-2);
Plättchen-entstammender Wachstumsfaktor (PDGF);
epidermaler Wachstumsfaktor (EGF);
Fibroblasten-Wachstumsfaktor (FGF);
Nerven-Wachstumsfaktor (NGF);
Kolonie-stimulierender Faktor (CSF);
transformierender Wachstumsfaktor (TGF);
Tumor-Nekrose-Faktor (TNF);
Calcitonin (CT);
Parathyreoidhormon (PTH);
Wachstumshormon (GH);
Östrogene;
Bombesin;
Knochenmorphogeneseprotein (bone morphogenetic protein, BMP);
Corticosteroide;
Insulin.

## Revendications

1. Une préparation pharmaceutique pour servir *in vivo* à obtenir une libération lente de facteurs de croissance et d'hormones, caractérisée en ce qu'elle est une combinaison de : une ou plusieurs protéines réceptrices/liantes pour lier les facteurs de croissance ou hormones, choisi dans le groupe consistant en récepteur de facteur 1 de croissance de type insuline ; récepteur de facteur 2 de croissance de type insuline ; récepteur d'insuline ; récepteur de facteur de croissance dérivé de plaquette ; récepteur de facteur de croissance de fibroblaste ; récepteur de facteur de croissance de l'épiderme ; récepteur de facteur de croissance de nerf ; récepteur de facteur de stimulateur de colonie ; récepteur de facteur de croissance transformant ; récepteur d'hormone de croissance ; récepteur d'hormone parathyroïdienne ; récepteur de calcitonine ; récepteur d'estrogène ; récepteur de facteur de nécrose de tumeur ; protéine de liaison sérique de facteur de croissance de type insuline ; globuline de liaison de corticostéroïde ; et de l'acide hyaluronique, ou ses dérivés, ou un polymère biodégradable choisi dans le groupe consistant en polyglycolide (PGA) ; copolymères de glycolide tel que copolymères glycolide/L-lactide (PGA/PLLA) ; copolymères glycolide/triméthylène carbonate (PGA/TMC) ; polylactides (PLA) ; stéréocopolymères de PLA comme poly-L-lactide (PLLA) ; poly-DL-lactide (PDLLA) ; copolymères L-lactide/DL-lactide ; copolymères de PLA tels que copolymères lactide/tetraméthylglycolide ; copolymères lactide/triméthylène carbonate ; copolymères lactide/α-valérolactone ; copolymères lactide/ε-caprolactone ; copolymères PLA/polyéthylène oxyde ; poly-1,4-dioxane-2,5-diones 3,6-substitués non symétriques ; poly-β-hydroxybutyrate (PHBA) ; copolymères PHBA/β-hydroxyvalérate (PHBA/HVA) ; poly-p-dioxanone (PDS) ; poly-δ-valérolactone ; poly-ε-caprolactone ; copolymères méthylméthacrylate-N-vinyle pyrrolidine ; polyestéramides ; polyesters d'acide oxalique ; polydihydropyranes ; polyalkyl-2-cyanoacrylates ; polyuréthanes (PU) ; polyvinylalcool (PVA) ; acide poly-β-malic (PMLA) ; acides poly-β-alcanoiques ;
en combinaison, avec les ligands aux protéines réceptrices de liaisons et en ce que les protéines réceptrices/de liaison sont mélangés, réticulés avec ou liés par covalence à l'acide hyaluronique ou au polymère biodégradable.

2. Préparation pharmaceutique selon la revendication 1, caractérisée en ce que les ligands sont par exemple : les facteurs de croissance 1 et 2 (IGF-1, IGF-2) de type insuline ; les facteurs de croissance dérivés de plaquette (PDGF) ; le facteur de croissance de l'épiderme (EGF) ; le facteur de croissance de fibroblaste (FGF) ; le facteur de croissance de nerf (NGF) ; le facteur stimulateur de colonie (CSF) ; le facteur de croissance transformant (TGF) ; le facteur de nécrose tumorale (TNF) ; la calcitonine (CT) ; l'hormone parathyroïdienne (PTH) ; l'hormone de croissance (GH) ; les estrogènes ; la bombésine ; la protéine morphogénétique osseuse (BMP) ; les corticostéroïdes ; l'insuline.
